# EUROPEAN PATENT APPLICATION

(11) **EP 2 770 092 A1**
(43) Date of publication of application: **27.08.2014**
(21) Application number: 13156729.9
(22) Date of filing: 26.02.2013
(51) Int. Cl.: D02G 3/44, D03D 1/00

(54) **Improved moisture-retaining and electrically conductive structure**

(71) Applicant: King's Metal Fiber Technologies Co., Ltd., Taichung City (TW)
(72) Inventor: Huang, Hong-Hsu, Taipei City (TW); Yang, Shun-Tung, Taipei City (TW); Peng, Jung-Hsiang, Taipei City (TW); Su, I-Chen, Taipei City (TW)
(74) Representative: Viering, Jentschura & Partner Patent- und Rechtsanwälte

(57) **Abstract**

An improved moisture-retaining and electrically conductive structure includes a plurality of moisture-retaining and electrically conductive yarns that is woven to form a planar structure, wherein each of the moisture-retaining and electrically conductive yarns is formed by blending a plurality of electrically conductive fibers and a plurality of moisture-retaining fibers. With such a structure, fabric of the same layer can provide both functions of moisture retention and electrical conduction and may improve the lifespan and electrical conductivity.

## Description

### FIELD OF THE INVENTION

The present invention relates to an improved moisture-retaining and electrically conductive structure, and in particular to an improved moisture-retaining and electrically conductive structure that provides both functions of moisture retention and electrical conduction.

### BACKGROUND OF THE INVENTION

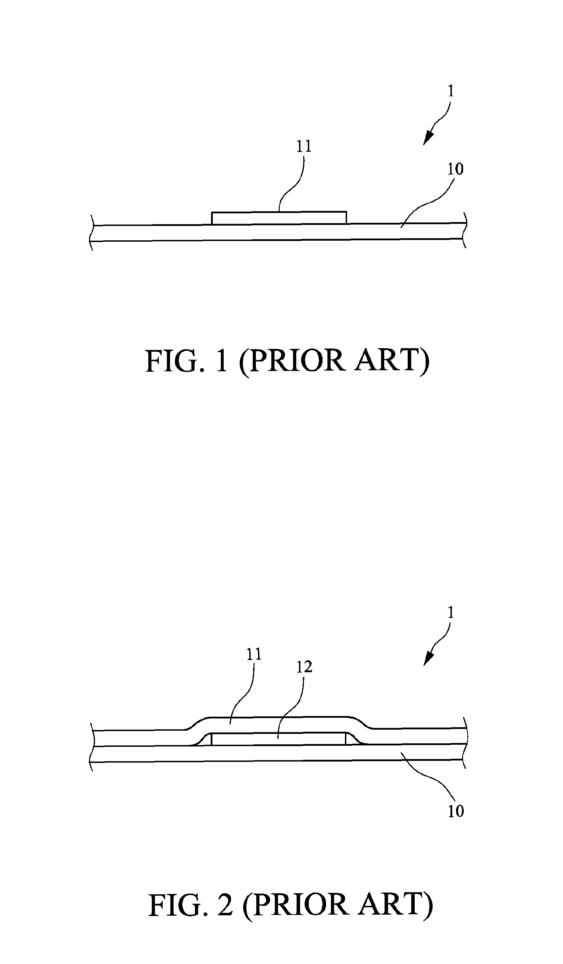

As shown in Figure 1, a conventional detection element 1 for physiological examination comprises a base layer 10 and an electrically conductive layer 11 formed on the base layer 10. To use, the electrically conductive layer is attached to human skin surface to detect a signal generated by the human body. However, in certain areas where the humidity is extremely low, electrical conduction between the electrically conductive layer 11 of the detection element 1 and the human skin is poor, making it difficult to detect the signal generated by the human body. As shown in Figure 2, an improvement is made such that a moisture-retaining layer 12 is arranged between the electrically conductive layer 11 and the base layer 10, wherein the moisture-retaining layer 12 functions to absorb and keep liquid therein in order to increase the humidity between the electrically conductive layer 11 and human skin and thus improve electrical conductivity of the electrically conductive layer 11. However, since the moisture-retaining layer 12 and the electrically conductive layer 11 are two separate layers, moisture must penetrate through the electrically conductive layer 11 before being absorbed by the moisture-retaining layer 12. Consequently, the absorbability of moisture is affected. When the moisture-retaining layer 12 releases water between the electrically conductive layer 11 and human skin, the release of water is also affected by being blocked by the electrically conductive layer 11. Further, since the moisture-retaining layer 12 and the electrically conductive layer 11 are two separate layers that are bonded to each other by an external force (such as adhesion). These layers are easily detached from each other due to the high humidity long maintained by the moisture-retaining layer 12, making the detection element 1 losing its function.

In view of this problem, the present invention aims to provide a structure that provides both functions of moisture retention and electrical conduction in order to achieve the goal of improving electrical conduction and lifespan of product.

### SUMMARY OF THE INVENTION

An object of the present invention is to provide an improved moisture-retaining and electrically conductive structure that provides both functions of moisture retention and electrical conduction.

To realize the above objectives, the present invention provides an improved moisture-retaining and electrically conductive structure, which comprises a plurality of moisture-retaining and electrically conductive yarns that is woven to form a planar structure, wherein each of the moisture-retaining and electrically conductive yarns is formed by blending a plurality of electrically conductive fibers and a plurality of moisture-retaining fibers.

In the moisture-retaining and electrically conductive structure discussed above, the electrically conductive fibers are one of metal fiber, carbon nanotube fiber, and carbon fiber.

In the moisture-retaining and electrically conductive structure discussed above, the moisture-retaining fibers are one of porous fiber, alginate fiber, carboxymethyl cellulose fiber, and rayon fiber.

In the moisture-retaining and electrically conductive structure discussed above, the moisture-retaining and electrically conductive yarns are woven through plain weaving to form the planar structure.

In the moisture-retaining and electrically conductive structure discussed above, the moisture-retaining and electrically conductive yarns are woven through knitting to form the planar structure.

### BRIEF DESCRIPTION OF THE DRAWINGS

The present invention will be apparent to those skilled in the art by reading the following description of preferred embodiments thereof with reference to the drawings, in which:

Figure 1 is a side elevational view showing a conventional detection element for physiological examination;

Figure 2 is a side elevational view showing a conventional detection element for physiological examination;

Figure 3 is a schematic view showing a moisture-retaining and electrically conductive yarn of an improved moisture-retaining and electrically conductive structure according to a first embodiment of the present invention;

Figure 4 is a schematic view showing the improved moisture-retaining and electrically conductive structure according to the first embodiment of the present invention; and

Figure 5 is a schematic view showing an improved moisture-retaining and electrically conductive structure according to a second embodiment of the present invention.

### DETAILED DESCRIPTION OF THE PREFERRED EMBODIMENTS

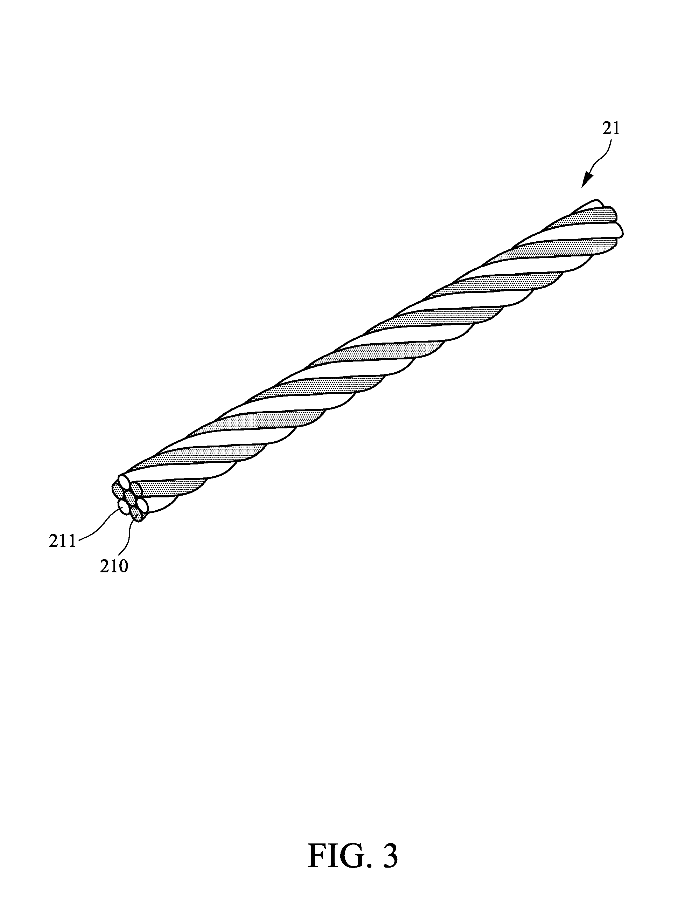

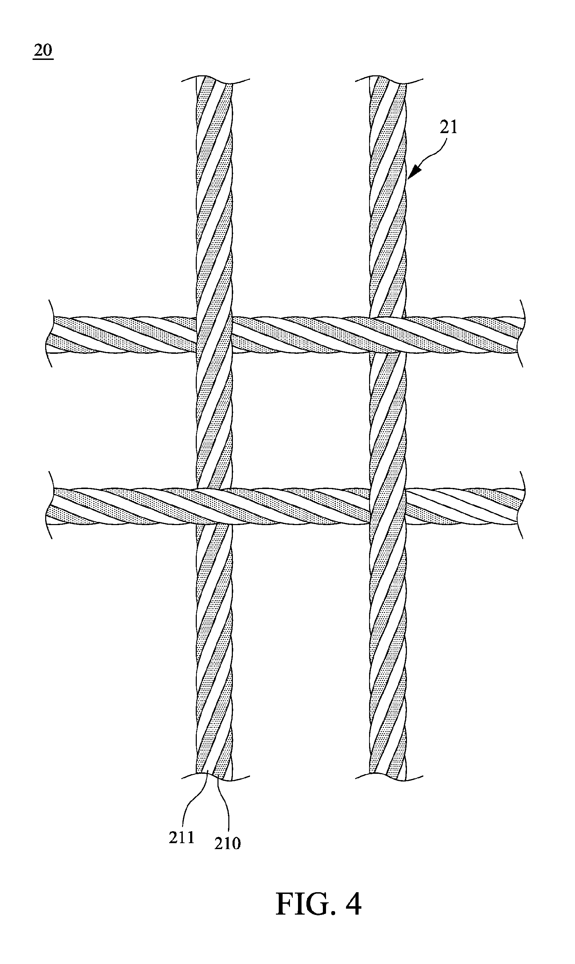

With reference to the drawings and in particular to Figures 3 and 4, of which Figure 3 is a schematic view showing a moisture-retaining and electrically conductive yarn of an improved moisture-retaining and electrically conductive structure according to a first embodiment of the present invention and Figure 4 is a schematic view showing the improved moisture-retaining and electrically conductive structure according to the first embodiment of the present invention, as shown in the drawings, in the instant embodiment, the improved moisture-retaining and electrically conductive structure 20 according to the present invention comprises a plurality of moisture-retaining and electrically conductive yarns 21. These moisture-retaining and electrically conductive yarns 21 are interlaced and woven through plain weaving to form a planar structure. Each of the moisture-retaining and electrically conductive yarns 21 is formed by blending electrically conductive fibers 210 and moisture-retaining fibers 211. The electrically conductive fiber 210 is one of metal fiber, carbon nanotube fiber, and carbon fiber. The moisture-retaining fiber 211 is one of porous fiber, alginate fiber, carboxymethyl cellulose fiber, and rayon fiber.

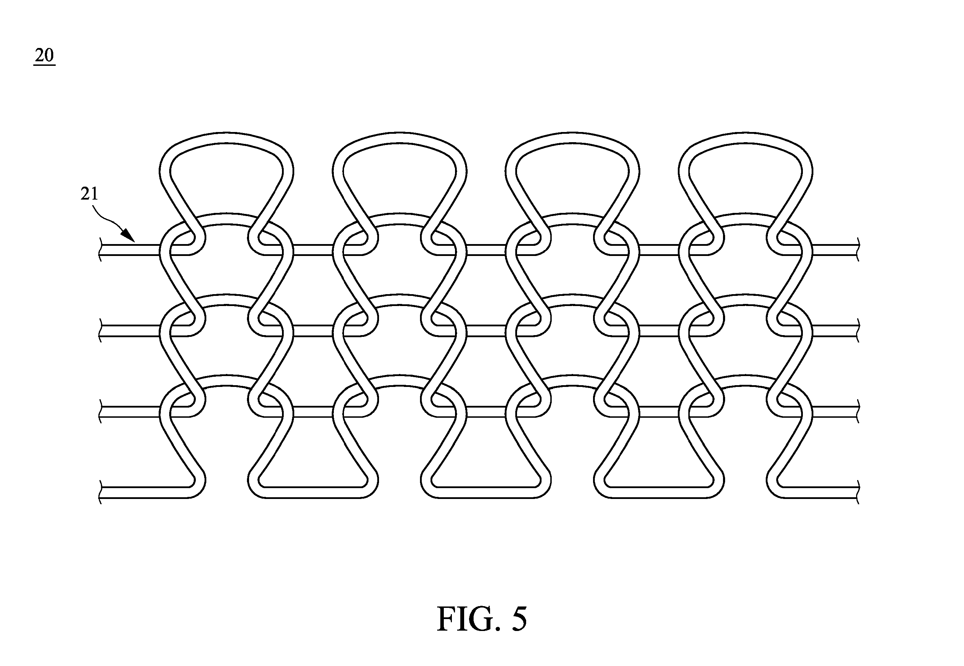

Referring to Figure 5, which is a schematic view showing an improved moisture-retaining and electrically conductive structure according to a second embodiment of the present invention, as shown in the drawing, in the instant embodiment, the moisture-retaining and electrically conductive yarns 21 of the improved moisture retaining and electrically conductive structure 20 according to the present invention are interlaced and woven through knitting to form a planar structure. The moisture-retaining and electrically conductive yarns 21 can be made of the same material as that discussed in the previous first embodiment.

The improved moisture-retaining and electrically conductive structure as discussed above are interlaced and woven to form a planar structure through plain weaving or knitting in order to allow the moisture-retaining and electrically conductive yarns 21 to directly absorb or release water to the space between the moisture-retaining and electrically conductive yarns 21 and human skin. Further, the moisture-retaining and electrically conductive yarns 21 are all set in the same layer and the influence on the functionality of electrical conduction and moisture retention by being long kept humid by the moisture-retaining yarns is eliminated. The present invention can effectively improve the shortcoming of the conventional device.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A moisture-retaining and electrically conductive structure, comprising:
a plurality of moisture-retaining and electrically conductive yarns that is woven to form a planar structure;
wherein each of the moisture-retaining and electrically conductive yarns is formed by blending a plurality of electrically conductive fibers and a plurality of moisture-retaining fibers.

2. The moisture-retaining and electrically conductive structure as claimed in Claim **1,** wherein the electrically conductive fibers are one of metal fiber, carbon nanotube fiber, and carbon fiber.

3. The moisture-retaining and electrically conductive structure as claimed in Claim **1,** wherein the moisture-retaining fibers are one of porous fiber, alginate fiber, carboxymethyl cellulose fiber, and rayon fiber.

4. The moisture-retaining and electrically conductive structure as claimed in Claim **1,** wherein the moisture-retaining and electrically conductive yarns are woven through plain weaving to form the planar structure.

5. The moisture-retaining and electrically conductive structure as claimed in Claim **1,** wherein the moisture-retaining and electrically conductive yarns are woven through knitting to form the planar structure.
